# EUROPÄISCHE PATENTSCHRIFT

(11) **EP 1 175 894 B1**
(45) Veröffentlichungstag und Bekanntmachung des Hinweises auf die Patenterteilung: **10.11.2004**
(21) Anmeldenummer: 01113597.7
(22) Anmeldetag: 15.06.2001
(51) Int. Cl.: A61K 7/135, C08G 77/455

(54) **Mittel zum Blondieren von menschlichen Haaren enthaltend Poly-(N-acylalkylenimin) modifizierte Organopolysikoxane**
Composition for bleaching human hair comprising poly-(n-acylalkyleneimine) modified organopolysiloxanes
Composition pour decolorer des cheveux humains comprenant des organopolysiloxanes modifies par poly-(n-acylalkyleneimine)

(30) Priorität: 19.06.2000 DE 10030095
(43) Veröffentlichungstag der Anmeldung: 30.01.2002
(73) Patentinhaber: KPSS-Kao Professional Salon Services GmbH, 64297 Darmstadt (DE)
(72) Erfinder: Nöcker, Bernd, Dr., 64372 Ober-Ramstadt (DE); Theis, Heinz, Dr., 64354 Reinheim (DE); Kure, Naohisa, Dr., Sumida-ku, Tokyo 131 (JP)

(56) Entgegenhaltungen:
- EP-A- 0 524 612
- EP-A- 0 842 656
- FR-A- 2 596 985
- US-A- 5 143 518
- US-A- 5 194 251

## Beschreibung

Die vorliegende Erfindung betrifft ein neues Mittel zum intensiven, jedoch gleichwohl schonenden Blondieren und Aufhellen von menschlichen Haaren.

Das Blondieren menschlicher Haare besteht üblicherweise aus folgenden Verfahrensschritten:

Homogenes Vermischen einer wasserfreien Zubereitung, vorzugsweise eines Pulvers, das mindestens eine Verbindung mit bleichender bzw. aufhellender Wirksamkeit, insbesondere ein festes Peroxidsalz, vorzugsweise Ammonium-, Kalium- und/oder Natriumpersulfat oder Erdalkaliperoxide enthält, mit einer wäßrigen Wasserstoffperoxid-Lösung, Aufbringen dieser Zusammensetzung auf das Haar, und Ausspülen nach vollendeter Blondierung. Dabei ist seit längerem bekannt, daß die Verwendung der im Hinblick auf die Blondierung wirksamsten Bestandteile, insbesondere der Persulfate, vor allem des Ammoniumpersulfats, in den erforderlichen höheren Konzentrationen zu Haarschädigungen führen kann.

Es wurde bereits versucht, dieses Problem durch (Teil-)Substitution der Persulfate zu lösen, was bisher jedoch nicht in zufriedenstellender Weise gelungen ist.

Es wurde nunmehr gefunden, und das ist Gegenstand der vorliegenden Erfindung, daß durch den Zusatz mindestens eines bestimmten Organopolysiloxans zu wasserfreie, insbesondere Peroxide als Wirkstoff enthaltenden Blondiermitteln überraschenderweise nicht nur eine verbesserte Blondierwirkung erreicht wird, ohne eine Haarschädigung hervorzurufen, sondern auch noch die Eigenschaften des so behandelten Haares insbesondere, die Naß- und Trockenkämmbarkeit sowie der Griff und das Volumen desselben verbessert werden.

Gegenstand der Erfindung ist auch ein Verfahren zum Blondieren von menschlichen Haaren durch Behandeln derselben mit einer Zubereitung, die durch Vermischen einer wasserfreien Zusammensetzung (A), die mindestens eine Verbindung mit bleichender bzw. aufhellender Wirksamkeit, vorzugsweise ein Peroxid, enthält, mit einer wäßrigen Wasserstoffperoxidzusammensetzung (B) erhalten wurde, wobei mindestens eine der Zusammensetzungen (A) und (B) mindestens ein bestimmtes Organopolysiloxan enthält.

Als Organopolysiloxan enthält die erfindungsgemäße Zusammensetzung ein solches, bei dem mindestens ein Siliciumatom über eine Alkylengruppe, die ein Heteroatom,insbesondere ein Stickstoffatom, aufweist, mit einem Poly-(N-acylalkylenimin) mit Einheiten der Formel I 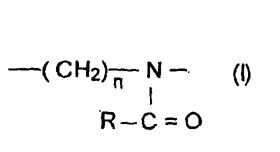 wobei n eine Zahl von 1 bis 5 und R Wasserstoff, eine C₁-C₁₂-Alkyl- oder -Cycloalkyl-, -Aralkyl- oder -Arylgruppe bedeuten, verbunden ist, vorzugsweise in einer Menge von 0,01 bis 5 Gew.-%, insbesondere 0,05 bis 2,5 Gew.-%, bezogen auf die Gesamtmenge des Mittels,

Bevorzugte Organopolysiloxan-Polymere sind solche, wie sie in der EP-A 524 612 beschrieben sind, insbesondere gegebenenfalls quatemierte Aminoalkyl-, insbesondere Aminopropyl-dimethylpolysiloxan/Polyethyloxazolin-Copolymerisate der Formel 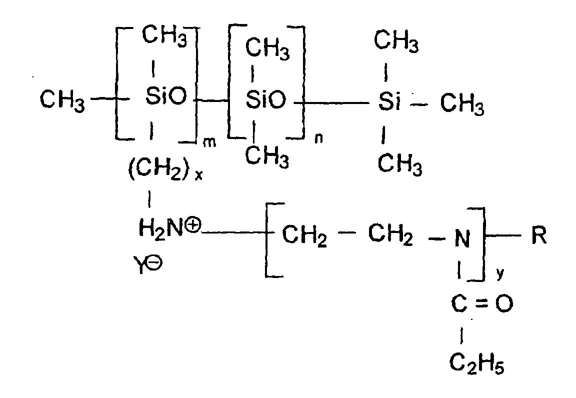 worin m und n jeweils ganze Zahlen von 20 bis 10 000, insbesondere 50 bis 7000, vor allem 100 bis 5000, x eine Zahl zwischen 1 und 5, vorzugsweise 3, und y eine Zahl von 5 bis 30 bedeuten, R eine C₁-C₁₂-Alkyl- oder Arylgruppe, insbesondere eine Methyl-, Ethyl- oder Benzylgruppe, und Y⁻ ein Anion darstellen.

Die Herstellung der erfindungsgemäß bevorzugt zum Einsatz gelangenden Aminoalkyldimethylpolysiloxan/Polyethyloxazolin-Pfropfcopolymerisate ist in der bereits erwähnten EP-A 524 612 beschrieben und erfolgt entsprechend dem folgenden Schema:

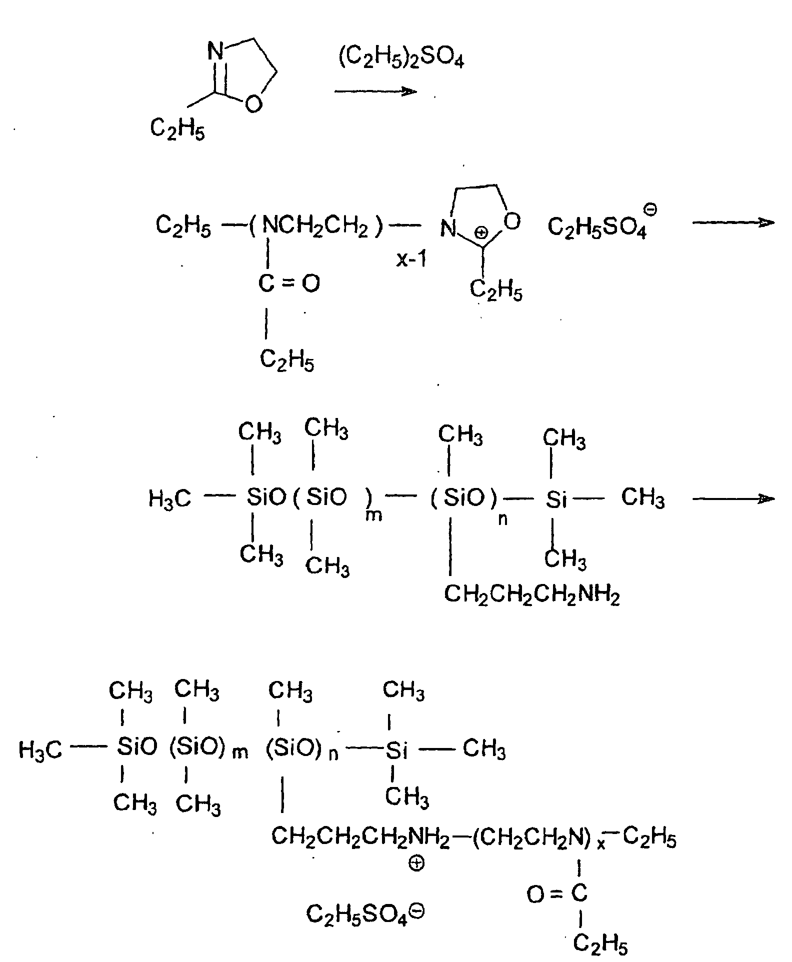

Das Anion Y⁻ der allgemeinen Formel kann selbstverständlich auch ein anderes als das Ethylsulfat-Anion des obengenannten Beispiels sein, d.h. die Quaternierung kann auch mit Methylchlorid, Dimethylsulfat, Benzylchlorid, Dodecylbromid etc. erfolgen.

Ein besonders bevorzugtes Pfropfcopolymerisat der dargestellten Art weist ein Gesamtmolekulargewicht von etwa 50 000 bis etwa 500 000, vorzugsweise etwa 80 000 bis etwa 300 000, insbesondere etwa 100 000 Dalton auf, wobei das Molgewicht des Oxazolin-Segments etwa 2 500 bis etwa 7 500, vorzugsweise etwa 4 000 bis etwa 6 000, insbesondere etwa 5 000 Dalton/Segment beträgt, d.h. der Molanteil bei 20 Einheiten/Molekül liegt. Der bevorzugte Si-Gehalt beträgt, entsprechend der Elementaranalyse, etwa 50 %.

Besonders geeignet sind die unter den Bezeichnungen A-1, A-2 und A-3 auf den Seiten 12 bis 13 der EP-A 640 643 beschriebenen Organopolysiloxane.
Der Anteil der Pfropfcopolymerisate in den erfindungsgemäßen Haarbehandlungsmitteln liegt bei etwa 0,05 bis 5, vorzugsweise 0,1 bis 2,5, insbesondere 0,5 bis 1,5 Gew.-% der Zusammensetzung.

Bei den wasserfreien Zusammensetzungen (A) handelt es sich vorzugsweise um Pulver, obwohl grundsätzlich auch Suspensionen bzw. Dispersionen der in der DE-A 38 14 356 beschriebenen Art einsetzbar sind.

Gemäß einer bevorzugten Ausführungsform der Erfindung werden die Blondier- und Aufhellungspulver als staubfreie Produkte verwendet, die als Granulate bzw. Agglomerate vorliegen.

Es kann sich dabei beispielsweise um die in der EP-A 560 088 beschriebenen Produkte, die ein Öl bzw. ein flüssiges Wachs enthalten, handeln, wobei das Aufbringen dieses Öls oder Wachses zweckmäßigerweise durch Aufsprühen erfolgt.

Auch staubfreie Blondierpulver, die entsprechend der EP-A 630 643 durch Agglomerieren der pulverförmigen Ausgangsbestandteile mittels Aufsprühen von geschmolzenen Wachsen oder C₁₀-C₁₈-Fettsäurealkanolamiden oder Verschmelzen mit denselben bei erhöhter Temperatur hergestellt wurden, sind erfindungsgemäß geeignet.

Neben dem aktiven Peroxid-Bestandteil enthalten die Blondiermittelzusammensetzungen noch die in solchen Mitteln üblichen Bestandteile:

Wenn es sich um ein Pulver handelt, insbesondere inerte pulverförmige Trägerstoffe, beispielsweise pyrogenes Siliciumioxid, Stärkepulver, etc., Alkalisierungsmittel wie Natriummetasilikat, oberflächenaktive Substanzen, Bindemittel, etc.; zur Vermeidung von Wiederholungen wird auf die einschlägigen Standardwerke, beispielsweise K. Schrader, "Grundlagen und Rezepturen der Kosmetika, 2. Auflage (1989, Hüthig Buchverlag), S.815 bis 823, verwiesen.

Als aktive Bestandteile werden Peroxide eingesetzt. Als solche sind insbesondere Persulfate wie Natrium- und Kaliumpersulfat, Ammoniumpersulfat, Erdalkaliperoxide wie Magnesiumperoxid, Melaminperoxid, Harnstoffperoxid oder Phtholimidoperoxyhexansäure.
Der Anteil an Peroxiden liegt bei mindestens 10, vorzugsweise mindestens 20 bis etwa 80 Gew.-%.
Gemäß einer bevorzugten Ausführungsform der Erfindung können die Blondiermittel noch 0,1 bis 10 Gew.-%, berechnet auf die Gesamtzusammensetzung, eines oder mehrerer Ammoniumsalze enthalten.

Geeignete Ammoniumsalze sind dabei Ammoniumcarbonat, Ammoniumhydrogencarbonat, Ammoniumcarbamat, Ammoniumchlorid, Ammoniumsulfat, Ammoniumphosphate, Ammoniumnitrat, Ammoniumbromid, Ammoniumjodid, Ammoniumthiosulfat, Ammoniummolybdat, Ammoniumvanadat, Ammoniumsulfamat, Ammoniumcitrat, Ammoniumsalicylat, Ammoniumvalerat, Ammoniumartrat, Ammoniumbenzoat, Ammoniumacetat, Ammoniumformiat und Ammoniumlactat.

Bevorzugt sind hierbei die Ammoniumphosphate wie NH₄H₂PO₄, (NH₄)₂HPO₄, (NH₄)₂NaPO₄, NaNH₄HPO₄ oder NH₄Na₂PO₄, Ammoniumchlorid, Ammoniumsulfat und Diammoniumhydrogencitrat sowie Ammoniumchlorid, vorzugsweise in einer Menge von 0,1 bis 10 Gew.-%.

Die Ammoniumverbindungen können auch, wie aus der EP 609 796 A2 bekannt, in entsprechend höheren Mengen als alleiniger Blondierwirkstoff verwendet werden.

Der Anteil der bleichend bzw. aufhellend wirksamen Verbindungen insgesamt liegt vorzugsweise zwischen etwa 5 und etwa 75, insbesondere etwa 25 und etwa 60 Gew.-%, bezogen auf das Aufhellungs- und Blondierpulver.

Die Teilchengrößen der erfindungsgemäßen Blondiermittel liegen in der Regel unterhalb 1 mm, vorzugsweise unterhalb 500 Mikron, beispielsweise bei weniger als 400 Mikron, insbesondere etwa 25 bis etwa 100 µm, was eine ausgezeichnete Verarbeitbarkeit, d.h. Mischbarkeit mit einer wäßrigen Wasserstoffperoxid-Lösung vor Applikation auf das menschliche Haar gewährleistet.

Die Anwendung des Mittels geschieht in an sich bekannter und üblicher Weise:

Das pulverförmige Aufhellungsmittel wird mit einer 6- bis 12-prozentigen Wasserstoffperoxid-Lösung, vorzugsweise in einem Verhältnis von etwa 1 Gewichtsteil des Pulvers zu etwa 0,5 bis etwa 4, insbesondere etwa 1 bis etwa 2,5 Gewichtsteilen der Peroxid-Lösung oder -Lotion homogen vermischt und anschließend etwa 10 bis etwa 60, insbesondere etwa 20 bis 30 Minuten, je nach Temperatur, auf dem Haar zur Einwirkung gebracht.

Die Anwendung kann auch in der Weise geschehen, daß das Blondierpulver und die Wassertoffperoxidlösung mit einer Cremegrundlage vermischt werden, und diese homogene Mischung dann auf das Haar aufgetragen wird.

Vorzugsweise ist die Zusammensetzung so eingestellt, daß beim Mischen mit wäßriger Wasserstoffperoxid-Lösung ein pH-Wert der gebrauchsfertigen Mischung von etwa 8 bis etwa 11,5, insbesondere zwischen 9 bis 11, erhalten wird.

Die erfindungsgemäß eingesetzten Organopolysiloxan-Pfropfcopolymerisate können auch der Wasserstoffperoxid-Zusammensetzung (B) zugesetzt werden.

Gegenstand der Erfindung ist daher auch ein Verfahren zum Blondieren und Aufhellen von menschlichen Haaren, wobei eine wasserfreie Zusammensetzung (A), die mindestens eine bleichend bzw. aufhellend wirkende Verbindung, insbesondere ein Peroxid enthält, mit einer wäßrigen Zusammensetzung (B), die Wasserstoffperoxid enthält, gemischt und die Mischung auf menschliches Haar aufgebracht und nach erfolgter Einwirkung aus dem Haar ausgespült wird, wobei mindestens eine der beiden Zusammensetzungen (A) und (B) mindestens ein Organopolysiloxan, bei dem mindestens ein Siliciumatom über eine Alkylgruppe, die ein Heteroatom, insbesondere ein Stickstoffatom, aufweist, mit einem Poly-(N-acylalkylenimin) mit Einheiten der Formel I 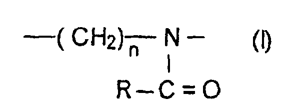 wobei n eine Zahl von 1 bis 5 und R Wasserstoff, eine C₁-C₁₂-Alkyl- oder -Cycloalkyl-, -Aralkyl- oder -Arylgruppe bedeuten, verbunden ist, enthält.

Die folgenden Beispiele dienen der Illustration der Erfindung.

### Beispiel 1

### a) Aufhellungspulver

| | |
|---|---|
| Siliciumdioxid | 15,8(Gew.-%) |
| Ammoniumchlorid | 14,7 |
| Natriumcarbonat | 10,0 |
| Natriumetasilicat | 2,3 |
| Phthalimidoperoxyhexansäure | 41,5 |
| Natriumpersulfat | 15,2 |
| Organopolysiloxan A-1 nach der | 0,5 |
| EP-A 640 643 | |

Diese Zusammensetzung wird im Gewichtsverhältnis 1:2,5:2,5 mit einer H₂O₂-Lotion b) und einer Cremegrundlage c) vermischt:

### b) Wasserstoffperoxid-Lotion

| | |
|---|---|
| Wasserstoffperoxid | 6,0 (Gew.-%) |
| Cetylstearylalkohol | 1,7 |
| Phosphorsäure | 0,5 |
| Natriumlaurylsulfat | 0,2 |
| Organopolysiloxan A-1 | 0,3 |
| nach der EP-A 640 643 | |
| Dinatriumhydrogenphosphat | 0,1 |
| Salicylsäure | 0,1 |
| Wasser | ad 100,0 |

### c) Cremegrundlage

| | |
|---|---|
| Cetylstearylalkohol | 11,0 (Gew.-%) |
| Oleth-5 | 5,0 |
| Ölsäure | 2,5 |
| Stearamide MEA | 2,3 |
| Cocoamide MEA | 2,3 |
| Natriumcetylstearylsulfat | 1,2 |
| 1,2-Propylenglykol | 1,0 |
| 1,2-Propylenglykolstearat | 0,6 |
| Natriumlaurylsulfat | 0,5 |
| Weizenproteinhydrolysat | 0,9 |
| Organopolysiloxan A-1 nach der | 0,4 |
| EP-A 640 643 | |
| Panthenol | 0,6 |
| Parfum | 0,4 |
| Komplexbildner | 0,2 |
| Wasser | ad 100,0 |

Der pH-Wert dieser Mischung lag bei 9,3.
Bei der Applikation dieser Mischung wurde eine ausgezeichnete, gleichmäßige Blondierwirkung festgestellt; eine feststellbare Haarschädigung trat an Haarsträhnen aus ungeschädigtem Menschenhaar selbst nach fünfmaliger aufeinanderfolgender Anwendung nicht auf.
Weglassen des Organopolysiloxans in den Zusammensetzungen führte zu einer verringerten, weniger gleichmäßigen Blondierwirkung, und einem weniger konditionierten Haar.

### Beispiel 2

| **Blondierpulver** | |
|---|---|
| Kieselgur | 36 (Gew.-Teile) |
| Natriumcarboxymethylcelluslose | 25 |
| Hydroxyethylcellulose | 16 |
| Natriumlaurylsulfat | 25 |
| Natriumstearat | 16 |
| Eiweißhydrolysat | 6 |
| Stärke | 10 |
| Natriumcarbonat | 8 |
| Natriummetasilikat | 90 |
| Polyvinylpyrrolidon K90 | 32 |
| Kokosfettsäuremonoethanolamid | 98 |
| (NH₄)₂HPO₄ | 20 |
| Magnesiumperoxid | 38 |
| Kaliumpersulfat | 80 |
| Natriumpersulfat | 500 |
| Organopolysiloxan A-1 nach der EP-A 640 643 | 10 |
| pH-Wert nach dem Anrühren mit 6%iger H₂O₂-Lösung im Verhältnis 1:1 | 9,5 |

Auch bei Anwendung dieser Mischung wurde eine ausgezeichnete, gleichmäßige Blondierwirkung, verbunden mit einer nicht meßbaren Haarschädigung, erhalten. Weglassen des Organopolysiloxans ergab einen deutlich verringerten Blondiereffekt.

### Beispiel 3

| **Staubfreies Blondierpulver** | |
|---|---|
| Siliciumdioxid (Diatomenerde) | 3,2 (Gew.-%) |
| Siliciumdioxid (pyrogenes SiO₂) | 5,3 |
| Natriumcarboxymethylcellulose | 3,5 |
| Harnstoff | 2,0 |
| Natriumlauroylsarkosinat | 0,8 |
| Natriumstearat | 1,2 |
| Natriumcarbonat | 1,0 |
| Natriummetasilikat | 6,0 |
| Stärkepulver | 3,5 |
| Kaliumpersulfat | 57,0 |
| Magnesiumperoxid | 4,0 |
| Organopolysiloxan A-2 nach der EP-A 640 643 | 1,0 |
| Paraffinöl (Paraffinum perliquidum, DAB 9) | 11,5 |

Es wird ein staubfreies Pulver erhalten, das mit einer bekannten 6%igen H₂O₂-Lösung im Gewichtsverhältnis 1:1 gut angerührt werden kann. 99% der Teilchen hatten einen Durchmesser von < 400 Mikron.

Die Blondierwirkung ist ausgezeichnet; eine Haarschädigung war nicht nachweisbar. Weglassen des Organopolysiloxans führte zu einer verringerten und ungleichmäßigeren Blondierwirkung; das Haar wies einen rauheren Griff und eine verringerte Naß- und Trockenkämmbarkeit auf.

Die Herstellung des Pulvers erfolgte durch Aufsprühen des Paraffinöls auf die Pulvergrundmasse bei etwa 20°C im Wirbelsprühverfahren.

### Beispiel 4

| **Staubfreies Blondier-Agglomerat** | |
|---|---|
| Siliciumdioxid (Diatomenerde) | 3,2 (Gew.-%) |
| Siliciumdioxid (pyrogenes SiO₂) | 5,3 |
| Natriumcarboxymethylcellulose | 3,5 |
| Harnstoff | 2,0 |
| Natriumlauroylsarkosinat | 0,8 |
| Natriumstearat | 1,2 |
| Natriumcarbonat | 1,0 |
| Natriummetasilikat | 6,0 |
| Stärkepulver | 3,5 |
| Natriumpersulfat | 39,5 |
| Kaliumpersulfat | 14,5 |
| Magnesiumperoxid | 4,0 |
| Cocosmonoethanolamid | 15,0 |
| Organopolysiloxan A-3 nach der EP-A 640 643 | 0,5 |

Es wurde ein staubfreies wasserlösliches bzw. -dispergierbares Pulver enthalten, das mit einer bekannten 9%igen H₂O₂-Lösung im Gewichtsverhältnis 1:1,5 gut angerührt werden konnte.
99% der Teilchen hatten einen Durchmesser von < 400 Mikron. Die am Haar erzielte Blondierwirkung war gleichmäßig ausgezeichnet; eine Haarschädigung war nicht erkennbar.
Die Herstellung des Pulvers erfolgte durch Aufheizen der obengenannten Mischung auf 70°C bis 75°C in einem Wirbelstromgenerator und anschließendes Abkühlen. Weglassen des Organopolysiloxan-Pfropfcopolymerisats führte zu einer verringerten Konditionier- und Blondierwirkung.

### Beispiel 5

| **Staubfreies Blondierpulver** | |
|---|---|
| Pyrogenes Silicia (Aerosil) | 2,0 (Gew.-Teile) |
| Hydroxyethylcellulose | 1,4 |
| Polyvinylpyrrolidon | 2,0 |
| Tetranatrium-EDTA | 2,0 |
| Natriumstearat | 1,3 |
| Natriumcarbonat | 1,0 |
| Ammoniumpersulfat | 8,0 |
| Magnesiumperoxid | 10,0 |
| Kaliumpersulfat | 40,0 |
| Natriummetasilikat | 11,5 |
| Natriumlaurylsulfat | 0,5 |
| Organopolysiloxan-Pfropfcopolymerisat | 1,0 |
| A-1 nach der EP-A 640 643 Aufgesprühtes Paraffinöl | 9,0 |

Das Pulver wurde mit einer wäßrigen 6%-igen H₂O₂-Zusammensetzung im Verhältnis 1:1 gemischt und die Mischung auf das Haar aufgebracht. Nach erfolgter Einwirkung, Waschen und Trocknen wurde ein gut und gleichmäßig blondiertes Haar mit dezentem Glanz, weichem Griff und guter Naß- und Trockenkämmbarkeit erhalten.

## Patentansprüche

1. Wasserfreies Mittel zum Blondieren von menschlichen Haaren, enthaltend
a) mindestens eine Verbindung mit bleichender bzw. aufhellender Wirkung, und
b) mindestens ein Organopolysiloxan, bei dem mindestens ein Siliciumatom über eire Alkylengruppe, die ein Heteroatom, insbesondere ein Stickstoffatom, aufweist, mit einem Poly-(N-acylalkylenimin) mit Einheiten der Formel I 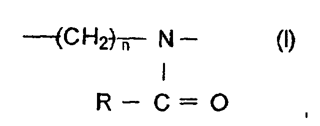
wobei n eine Zahl von 1 bis 5 und R Wasserstoff, eine C₁-C₁₂-Alkyl-, Cycloalkyl-, Aralkyl- oder Arylgruppe bedeuten, verbunden ist.

2. Mittel nach Anspruch 1, enthaltend als Organosiloxan ein gegebenenfalls quaterniertes Aminoalkyldimethylpolysiloxan/Polyethyloxazolin-Copolymerisat der Formel 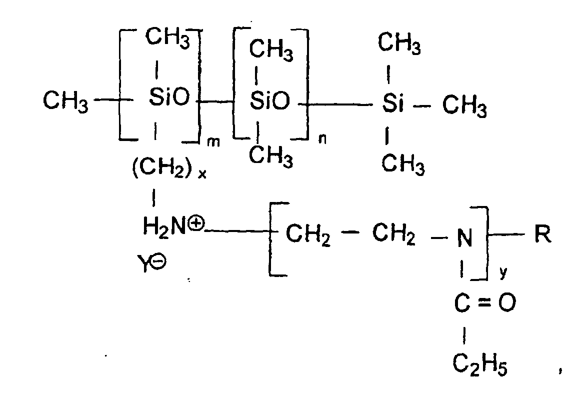 worin m und n jeweils ganze Zahlen von 20 bis 10 000, insbesondere 50 bis 7000, vor allem 100 bis 5000, x eine Zahl zwischen 1 und 5, vorzugsweise 3, und y eine Zahl von 5 bis 30 bedeuten, R eine C₁-C₁₂-Alkyl- oder Arylgruppe, insbesondere eine Methyl-, Ethyl- oder Benzylgruppe, und Y⁻ ein Anion darstellen.

3. Mittel nach Anspruch 1 oder 2, enthaltend 0,05 bis 5 Gew.-%, berechnet auf die Gesamtzusammensetzung, des Organopolysiloxans.

4. Mittel nach Anspruch 3, enthaltend 0,1 bis 2,5 Gew.-% des Organopolysiloxans, berechnet auf die Gesamtzusammensetzung des Mittels.

5. Mittel nach einem oder mehreren der Ansprüche 1 bis 4, enthaltend als Verbindung mit bleichender bzw. aufhellender Wirkung mindestens ein Peroxid.

6. Mittel nach einem oder mehreren der Ansprüche 1 bis 5, enthaltend als Verbindung mit bleichender bzw. aufhellender Wirkung mindestens ein Ammoniumsalz.

7. Verfahren zum Blondieren und Aufhellen von menschlichen Haaren, wobei eine wasserfreie Zusammensetzung (A), die mindestens eine bleichend bzw. aufhellende Verbindung enthält, mit einer wäßrigen Wasserstoffperoxidzusammensetzung (B) vermischt und die Mischung auf menschliches Haar aufgebracht wird, wobei mindestens eine der Zusammensetzungen (A) und (B) mindestens ein Organopolysiloxan, bei dem mindestens ein Siliciumatom über eine Alyklgruppe, die ein Heteroatom, insbesondere ein Stickstoffatom, aufweist, mit einem Poly-(N-acylalkylenimin) mit Einheiten der Formel I 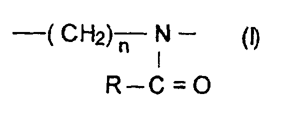 wobei n eine Zahl von 1 bis 5 und R Wasserstoff, eine C₁-C₁₂-Alkyl- oder- Cycloalkyl-, -Aralkyl- oder -Arylgruppe bedeuten, verbunden ist, enthält.

## Claims

1. Water-free composition for the bleaching of human hair, comprising
a) at least one compound with a bleaching or brightening effect, and
b) at least one organopolysiloxane, wherein at least one silicium atom is linked by an alkylene group having a hetero-atom, in particular a nitrogen atom, to a poly-(N-acyl alkyleneimine) with units of the formula I 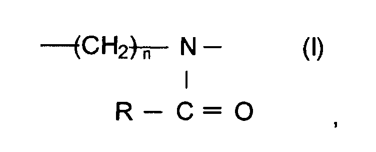
wherein n is a number from 1 to 5, and R is hydrogen, a C₁-C₁₂-alkyl, cycloalkyl, aralkyl or aryl group.

2. Composition according to claim 1, containing as organosiloxane an optionally quaternized aminoalkyl dimethyl polysiloxane/polyethyl oxazolines copolymer of the formula 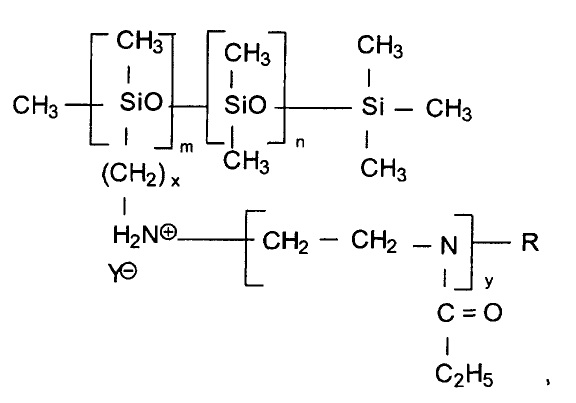 wherein m and n are each numbers from 20 to 10,000, in particular 50 to 7,000, especially 100 to 5,000, x is a number between 1 and 5, preferably 3, and y is a number from 5 to 30, R is a C₁-C₁₂-alkyl or aryl group, in particular a methyl, ethyl or benzyl group, and Y⁻ is an anion.

3. Composition according to claim 1 or 2, containing 0.05 % to 5 % by weight, calculated to the total composition, of the organopolysiloxane.

4. Composition according to claim 3, containing 0.1 % to 2.5 % by weight, calculated to the total composition, of the organopolysiloxanes.

5. Composition according to one or more of claims 1 to 4, containing as compound with the bleaching or brightening effect at least one peroxide.

6. Composition according to one or more of claims 1 to 5, containing as compound with bleaching or brightening effect at least one ammonium salt.

7. Process for the bleaching and high-lifting of human hair, wherein a water-free composition (A), containing at least one bleaching or high-lifting compound, is mixed with an aqueous hydrogen peroxide composition (B) and the mixture is applied onto human hair, whereby at least one the compositions (A) and (B) contains at least one organopolysiloxane, wherein at least one silicium atom is linked by an alkyl group having a hetero-atom, in particular a nitrogen atom, to a poly-(N-acyl alkyleneimine) with units of the formula I 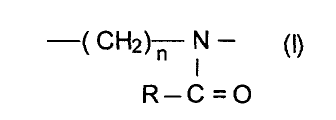 wherein n is a number from 1 to 5, and R is hydrogen, a C₁-C₂-alkyl or cycloalkyl, aralkyl or aryl group.

## Revendications

1. Agent exempt d'eau pour blondir des cheveux humains, qui contient:
a) au moins un composé à effet décolorant ou éclaircissant, et
b) au moins un organopolyxilosane dans lequel au moins un atome de silicium est relié par l'intermédiaire d'un groupe alkylène qui présente un hétéroatome, en particulier un atome d'azote, à un poly(N-acylalkylènimine) qui contient des unités de formule I 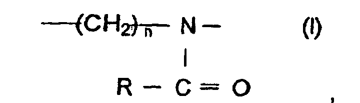
dans laquelle n représente un nombre de 1 à 5 et R représente l'hydrogène, ou un groupe C₁-C₁₂-alkyle, cycloalkyle, aralkyle ou aryle.

2. Agent selon la revendication 1, qui contient comme organoxilosane un copolymère éventuellement quaternisé d'aminoalkyldiméthylpolysiloxane et de poléthyloxazoline de formule: 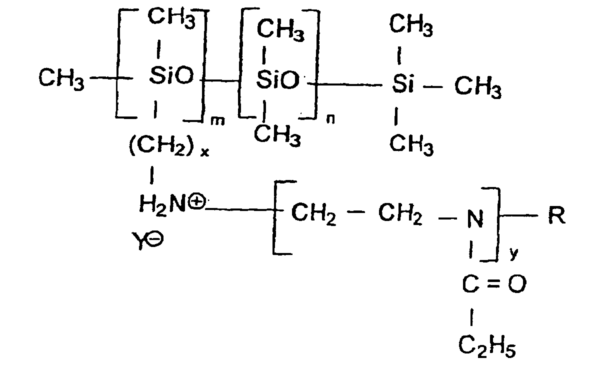 dans laquelle m et n représentent tous deux des nombres entiers compris entre 20 et 10 000, en particulier entre 50 et 7 000 et surtout entre 100 et 5 000, x un nombre compris entre 1 et 5, de préférence 3 et y un nombre compris entre 5 et 30, R représentant un groupe C₁-C₁₂-alkyle ou aryle et en particulier un groupe méthyle, éthyle ou benzyle et un Y⁻ représente un anion.

3. Agent selon les revendications 1 ou 2, qui contient de 0,05 à 5 % en poids de l'organopolysiloxane par rapport à la composition totale.

4. Agent selon la revendication 3, qui contient de 0,1 à 2,5% en poids de l'organopolysiloxane par rapport à la composition totale de l'agent.

5. Agent selon l'une ou plusieurs des revendications 1 à 4, qui contient comme composé à effet décolorant ou éclaircissant au moins un peroxyde.

6. Agent selon l'une ou plusieurs des revendications 1 à 5, qui contient comme composé à effet décolorant ou éclaircissant au moins un sel d'ammonium.

7. Procédé pour blondir et éclaircir des cheveux humains, dans lequel une composition (A) exempte d'eau qui contient au moins un composé décolorant ou éclaircissant est mélangé avec une composition (B) aqueuse de peroxyde d'hydrogène, le mélange étant appliqué sur des cheveux humains, au moins l'une des compositions (A) et (B) contenant au moins un organopolyxilosane dans lequel au moins un atome de silicium est relié par un groupe alkyle qui présente un hétéroatome, en particulier un atome d'azote, à un poly(N-acylalkylènimine) qui contient des unités de formule I 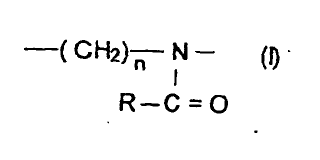 dans laquelle n représente un nombre de 1 à 5 et R représente l'hydrogène ou un groupe C₁-C₁₂-alkyle, -cycloalkyle, -aralkyle ou -aryle.
